# EUROPÄISCHE PATENTSCHRIFT

(11) **EP 0 906 291 B1**
(45) Veröffentlichungstag und Bekanntmachung des Hinweises auf die Patenterteilung: **09.03.2005**
(21) Anmeldenummer: 97923907.6
(22) Anmeldetag: 15.05.1997
(51) Int. Cl.: C07D 239/54, A01N 43/54

(54) **SUBSTITUIERTE PHENYLURACILE**
SUBSTITUTED PHENYL URACILS
PHENYLURACILES SUBSTITUES

(30) Priorität: 28.05.1996 DE 19621311
(43) Veröffentlichungstag der Anmeldung: 07.04.1999
(73) Patentinhaber: Bayer CropScience AG, 40789 Monheim (DE)
(72) Erfinder: DREWES, Mark, Wilhelm, D-40764 Langenfeld (DE); ANDREE, Roland, D-40764 Langenfeld (DE); DOLLINGER, Markus, Kansas 66213/US (DE)
(86) Internationale Anmeldenummer: PCT/EP1997/002488
(87) Internationale Veröffentlichungsnummer: WO 1997/045418

(56) Entgegenhaltungen:
- EP-A- 0 542 685
- WO-A-93/06090
- WO-A-95/25725
- DE-A- 4 237 920

## Beschreibung

Die Erfindung betrifft neue substituierte Phenyluracile, Verfahren zu ihrer Herstellung und ihre Verwendung als Herbizide.

Es ist bekannt, daß bestimmte substituierte Uracile herbizide Eigenschaften aufweisen (vgl. DE 4131038, DE 4237920, DE 4329537, EP 408382/US 5084084/US 5127935/US5154755, EP 542685, EP 563384, EP 648749, US 4979982, US 5169430, WO 91/00278, WO 95/25725). Diese Verbindungen haben jedoch bisher keine nennenswerte Bedeutung erlangt.

Es wurden nun die neuen substituierten Phenyluracile der allgemeinen Formel (I) gefunden 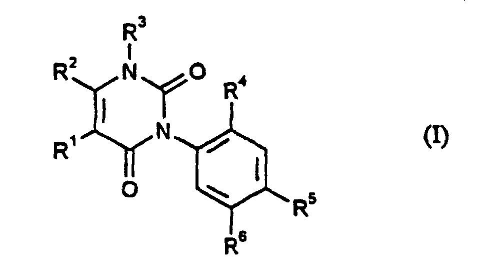
- R¹: für Wasserstoff, Fluor, Chlor, Brom oder gegebenenfalls durch Fluor und/oder Chlor substituiertes C₁-C₄-Alkyl steht,
- R²: für gegebenenfalls durch Fluor und/oder Chlor substituiertes C₁-C₄-Alkyl steht,
- R³: für Wasserstoff, Amino, für gegebenenfalls durch Cyano, Fluor, Chlor oder C₁-C₄-Alkoxy substituiertes C₁-C₆-Alkyl oder für gegebenenfalls durch Fluor und/oder Chlor substituiertes C₂-C₆-Alkenyl oder C₂-C₆-Alkinyl steht,
- R⁴: für Wasserstoff, Cyano, Fluor oder Chlor steht,
- R⁵: für Cyano oder Thiocarbamoyl steht, und
- R⁶: für eine der nachstehenden Gruppierungen steht 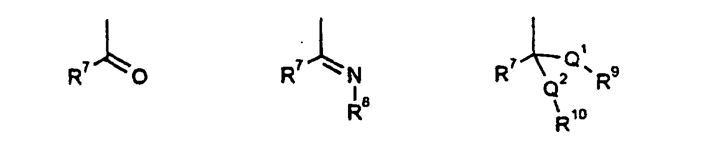
worin
- Q¹ und Q²: für Sauerstoff oder Schwefel stehen,
- R⁷: jeweils für Wasserstoff oder für gegebenenfalls durch Halogen oder C₁-C₄-Alkoxy substituiertes C₁-C₄-Alkyl steht,
- R⁸: für Wasserstoff, Hydroxy, Amino, für jeweils gegebenenfalls durch Halogen, C₁-C₄-Alkoxy oder C₁-C₄-Alkoxy-carbonyl substituiertes C₁-C₆-Alkyl, C₁-C₆-Alkoxy, C₁-C₆-Alkylamino, Di-(C₁-C₄-alkyl)-amino, C₁-C₄-Alkylcarbonylamino oder C₁-C₄-Alkyl-sulfonylamino, für jeweils gegebenenfalls durch Halogen substituiertes C₂-C₆-Alkenyl, C₂-C₆-Alkenyloxy oder C₂-C₆-Alkinyl, für jeweils gegebenenfalls durch Halogen oder C₁-C₄-Alkyl substituiertes C₃-C₆-Cycloalkyl, C₃-C₆-Cycloalkyloxy, C₃-C₆-Cycloalkylamino, C₃-C₆-Cycloalkyl-carbonylamino oder C₃-C₆-Cycloalkylsulfonylamino, oder für jeweils gegebenenfalls durch Halogen, C₁-C₄-Alkyl, C₁-C₄-Halogenalkyl, C₁-C₄-Alkoxy oder C₁-C₄-Halogenalkoxy substituiertes Phenyl, Phenoxy, Phenylamino, Phenylcarbonylamino, Phenylsulfonylamino, Phenyl-C₁-C₄-alkyl, Phenyl-C₁-C₄-alkoxy, Phenyl-C₁-C₄-alkylamino steht, sowie
- R⁹ und R¹⁰: für C₁-C₄-Alkyl stehen.

Man erhält die neuen substituierten Phenyluracile der allgemeinen Formel (I), wenn man
(a) Alkenylphenyluracile der allgemeinen Formel (II) 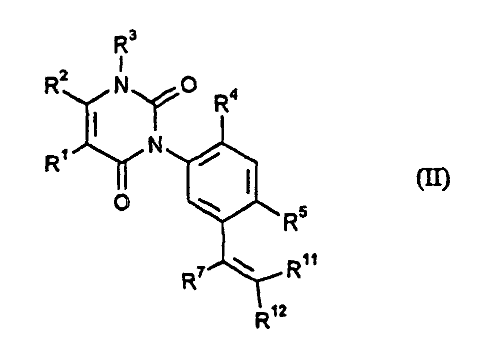 in welcher
   - R¹, R², R³, R⁴, R⁵ und R⁷: die oben angegebenen Bedeutungen haben und
   - R¹¹ und R¹²: für Cyano, Carboxy oder für C₁-C₄-Alkoxycarbonyl stehen
   mit Ozon gegebenenfalls in Gegenwart eines Verdünnungsmittels und gegebenenfalls in Gegenwart eines Reaktionshilfsmittels umsetzt,
   oder wenn man
(b) Carbonylphenyluracile der allgemeinen Formel (Ia) 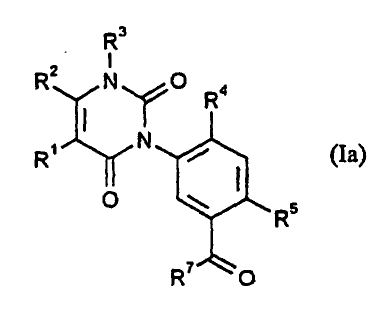 in welcher
   - R¹, R², R³, R⁴, R⁵ und R⁷: die oben angegebenen Bedeutungen haben,
   mit Aminoverbindungen der allgemeinen Formel (III)

   H₂N-R⁸ (III)

   in welcher
   - R⁸: die oben angegebene Bedeutung hat,
   - oder mit Säureaddukten von Verbindungen der Formel (III) -
   gegebenenfalls in Gegenwart eines Verdünnungsmittels und gegebenenfalls in Gegenwart eines Reaktionshilfsmittel umsetzt,
   oder wenn man
(c) Carbonylphenyluracile der allgemeinen Formel (Ia) 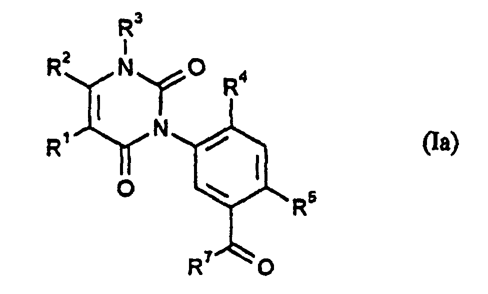 in welcher
   - R¹, R², R³, R⁴, R⁵ und R⁷: die oben angegebenen Bedeutungen haben,
   mit Alkoholen und/oder Mercaptanen der allgemeinen Formeln (IVa) oder (IVb)

   HQ¹R⁹ (IVa)

   HQ²R¹⁰ (IVb)

   worin
   - Q¹, Q², R⁹ und R¹⁰: die oben angegebenen Bedeutungen haben,
   gegebenenfalls in Gegenwart eines Verdünnungsmittels und gegebenenfalls in Gegenwart eines Reaktionshilfsmittel umsetzt.

Die Verbindungen der allgemeinen Formel (I) können auch nach weiteren üblichen Methoden in andere Verbindungen der allgemeinen Formel (I) gemäß obiger Definition umgewandelt werden, beispielsweise durch Aminierung bzw. Alkylierung (z.B. R³: H → NH₂, H → CH₃), Umsetzung mit Hydrogensulfid (z.B. R⁵: CN → CSNH₂).

Die neuen substituierten Phenyluracile der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- R¹: für Wasserstoff, Fluor, Chlor, Brom oder gegebenenfalls durch Fluor und/oder Chlor substituiertes Methyl oder Ethyl steht,
- R²: für gegebenenfalls durch Fluor und/oder Chlor substituiertes Methyl oder Ethyl steht,
- R³: für Wasserstoff, Amino, für gegebenenfalls durch Cyano, Fluor, Chlor, Methoxy oder Ethoxy substituiertes Methyl, Ethyl, n- oder i-Propyl, n-, i-, s- oder t-Butyl, oder für gegebenenfalls durch Fluor und/oder Chlor substituiertes Propenyl, Butenyl, Propinyl oder Butinyl steht,
- R⁴: für Wasserstoff, Fluor oder Chlor steht,
- R⁵: für Cyano oder Thiocarbamoyl steht, und
- R⁶: für eine der nachstehenden Gruppierungen steht 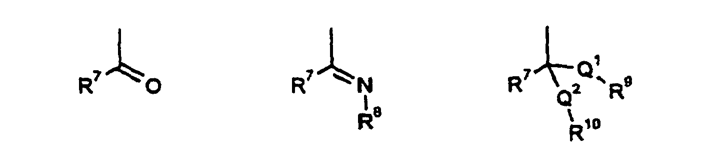
worin
- Q¹ und Q²: für Sauerstoff oder Schwefel stehen,
- R⁷: jeweils für Wasserstoff oder für jeweils gegebenenfalls durch Fluor, Chlor, Methoxy oder Ethoxy substituiertes Methyl oder Ethyl steht,
- R⁸: für Wasserstoff, Hydroxy, Amino, für jeweils gegebenenfalls durch Fluor, Chlor, Methoxy, Ethoxy, Methoxycarbonyl oder Ethoxycarbonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Acetylamino, Propionylamino, n- oder i-Butyroylamino, Methylsulfonylamino oder Ethylsulfonylamino, für jeweils gegebenenfalls durch Fluor, Chlor oder Brom substituiertes Propenyl, Butenyl, Propenyloxy, Butenyloxy, Propinyl oder Butinyl, für jeweils gegebenenfalls durch Fluor, Chlor, Brom, Methyl oder Ethyl substituiertes Cyclopentyl, Cyclohexyl, Cyclopentyloxy, Cyclohexyloxy, Cyclopentylamino, Cyclohexylamino, Cyclopropylcarbonylamino, Cyclobutylcarbonylamino, Cyclopentylcarbonylamino, Cyclohexylcarbonylamino, Cyclopropylsulfonylamino, Cyclobutylsulfonylamino, Cyclopentylsulfonylamino oder Cyclohexylsulfonylamino, oder für jeweils gegebenenfalls durch Fluor, Chlor, Brom, Methyl, Ethyl, Trifluormethyl, Methoxy, Ethoxy, Difluormethoxy oder Trifluormethoxy substituiertes Phenyl, Phenylamino, Phenylcarbonylamino, Phenylsulfonylamino, Benzyl, Benzyloxy oder Benzylamino steht, sowie
- R⁹ und R¹⁰: für Methyl oder Ethyl stehen.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

### Gruppe 1

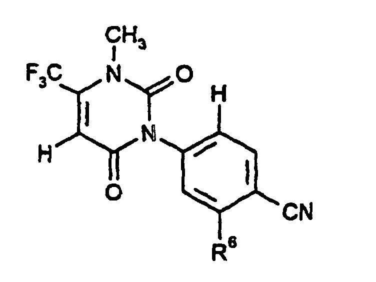

- R⁶: hat hierbei beispielhaft die in der nachstehenden Aufzählung angegebenen Bedeutungen:
Formyl, Acetyl, Methyliminomethyl, Ethyliminomethyl, n-Propyliminomethyl, i-Propyliminomethyl, Hydroximinomethyl, Methoximinomethyl, Ethoxyiminomethyl, n-Propoximinomethyl, i-Propoximinomethyl, n-Butoximinomethyl, 1-Methoximino-ethyl, Methoxycarbonylmethoximinomethyl, Ethoxycarbonylmethoximinomethyl, 1-Methoxycarbonylmethoximino-ethyl, 1-Ethoxycarbonylmethoximino-ethyl, Hydraziminomethyl, Methylhydraziminomethyl, Dimethylhydraziminomethyl, Acetylhydraziminomethyl, Methylsulfonylhydraziminomethyl, Allyloximinomethyl, Cyclopentyloximinomethyl, Cyclohexyloximinomethyl, Benzyloximinomethyl, Cyclopentylhydraziminomethyl, Cyclohexylhydraziminomethyl, Phenylhydraziminomethyl, Phenylcarbonylhydraziminomethyl, Phenylsulfonylhydraziminomethyl, Benzylhydraziminomethyl, Dimethoxymethyl, Diethoxymethyl.

### Gruppe 2

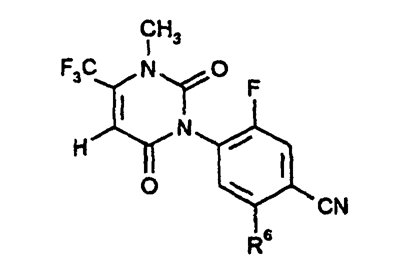

- R⁶: hat hierbei beispielhaft die oben in Gruppe 1 aufgeführten Bedeutungen.

### Gruppe 3

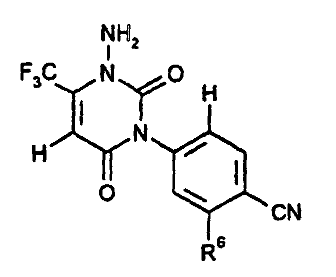

- R⁶: hat hierbei beispielhaft die oben in Gruppe 1 aufgeführten Bedeutungen.

### Gruppe 4

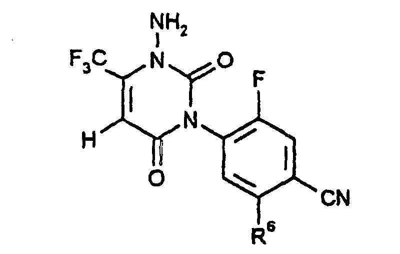

- R⁶: hat hierbei beispielhaft die oben in Gruppe 1 aufgeführten Bedeutungen.

### Gruppe 5

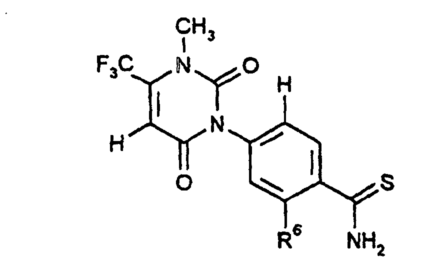

- R⁶: hat hierbei beispielhaft die oben in Gruppe 1 aufgeführten Bedeutungen.

### Gruppe 6

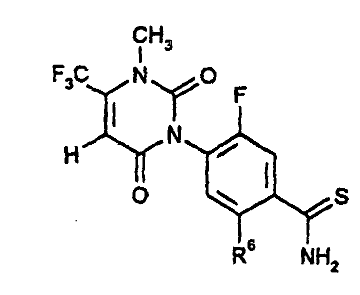

- R⁶: hat hierbei beispielhaft die oben in Gruppe 1 aufgeführten Bedeutungen.

### Gruppe 7

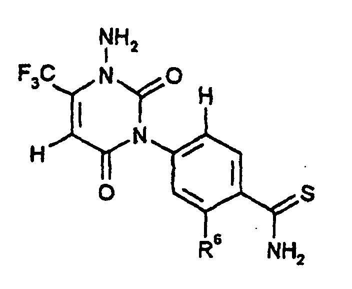

- R⁶: hat hierbei beispielhaft die oben in Gruppe 1 aufgeführten Bedeutungen.

### Gruppe 8

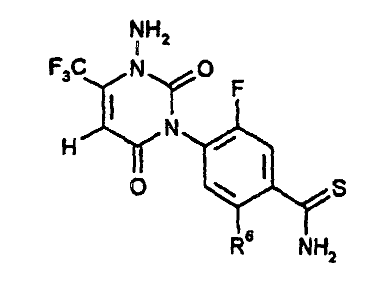

- R⁶: hat hierbei beispielhaft die oben in Gruppe 1 aufgeführten Bedeutungen.

### Gruppe 9

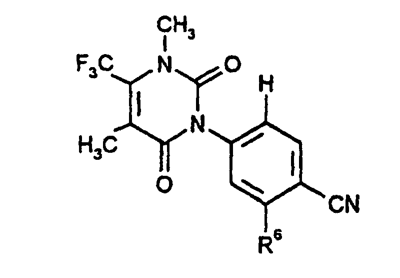

- R⁶: hat hierbei beispielhaft die oben in Gruppe 1 aufgeführten Bedeutungen.

### Gruppe 10

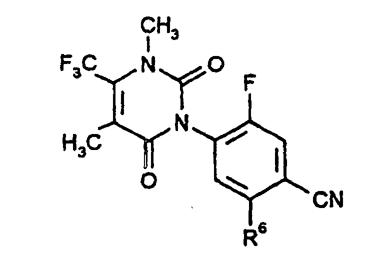

- R⁶: hat hierbei beispielhaft die oben in Gruppe 1 aufgeführten Bedeutungen.

### Gruppe 11

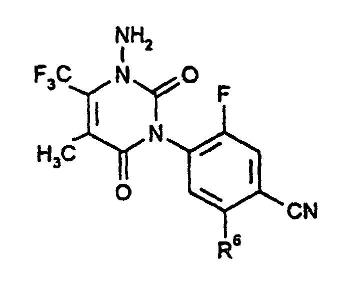

- R⁶: hat hierbei beispielhaft die oben in Gruppe 1 aufgeführten Bedeutungen.

### Gruppe 12

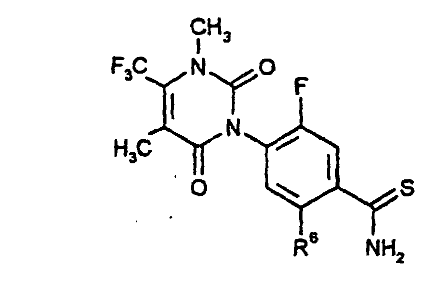

- R⁶: hat hierbei beispielhaft die oben in Gruppe 1 aufgeführten Bedeutungen.

### Gruppe 13

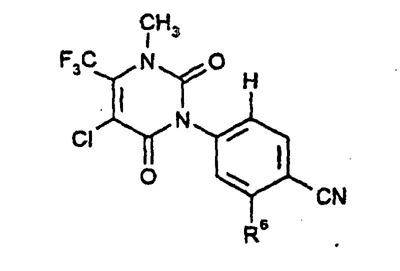

- R⁶: hat hierbei beispielhaft die oben in Gruppe 1 aufgeführten Bedeutungen.

### Gruppe 14

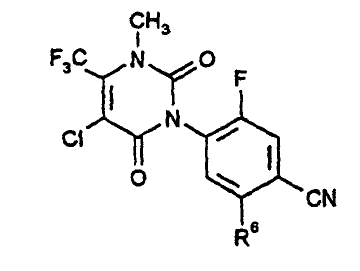

- R⁶: hat hierbei beispielhaft die oben in Gruppe 1 aufgeführten Bedeutungen.

### Gruppe 15

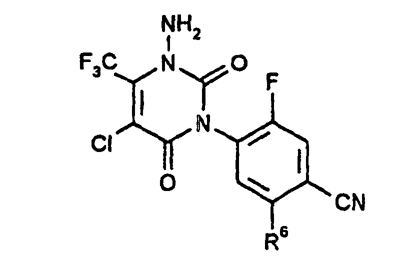

- R⁶: hat hierbei beispielhaft die oben in Gruppe 1 aufgeführten Bedeutungen.

### Gruppe 16

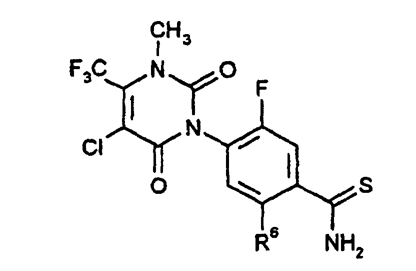

- R⁶: hat hierbei beispielhaft die oben in Gruppe 1 aufgeführten Bedeutungen.

### Gruppe 17

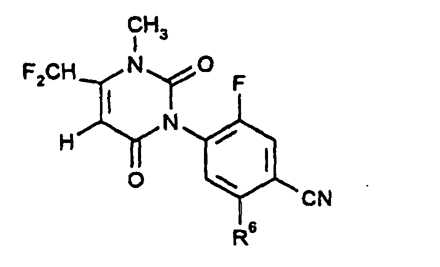

- R⁶: hat hierbei beispielhaft die oben in Gruppe 1 aufgeführten Bedeutungen.

### Gruppe 18

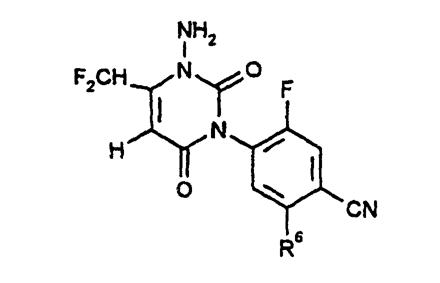

- R⁶: hat hierbei beispielhaft die oben in Gruppe 1 aufgeführten Bedeutungen.

### Gruppe 19

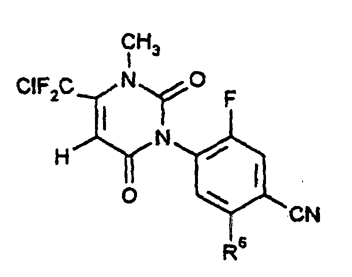

- R⁶: hat hierbei beispielhaft die oben in Gruppe 1 aufgeführten Bedeutungen.

### Gruppe 20

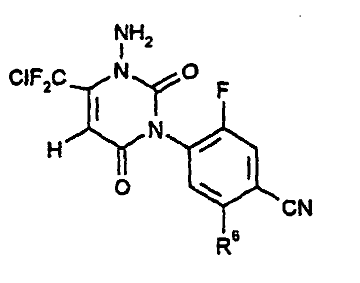

- R⁶: hat hierbei beispielhaft die oben in Gruppe 1 aufgeführten Bedeutungen.

### Gruppe 21

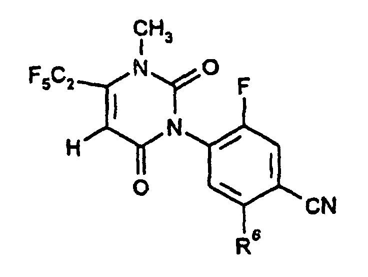

- R⁶: hat hierbei beispielhaft die oben in Gruppe 1 aufgeführten Bedeutungen.

### Gruppe 22

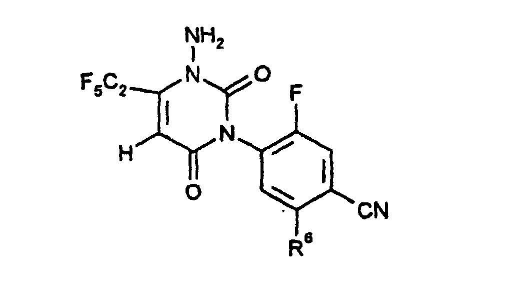

- R⁶: hat hierbei beispielhaft die oben in Gruppe 1 aufgeführten Bedeutungen.

Verwendet man beispielsweise 1-[4-Cyano-2-fluor-5-(2-methoxycarbonyl-ethenyl)-phenyl]-3,6-dihydro-2,6-dioxo-3-methyl-4-trifluormethyl-1(2H)-pyrimidin und Ozon als Ausgangsstoffe, so kann der Reaktionsablauf beim erfindungsgemäßen Verfahren (a) durch das folgende Formelschema skizziert werden:

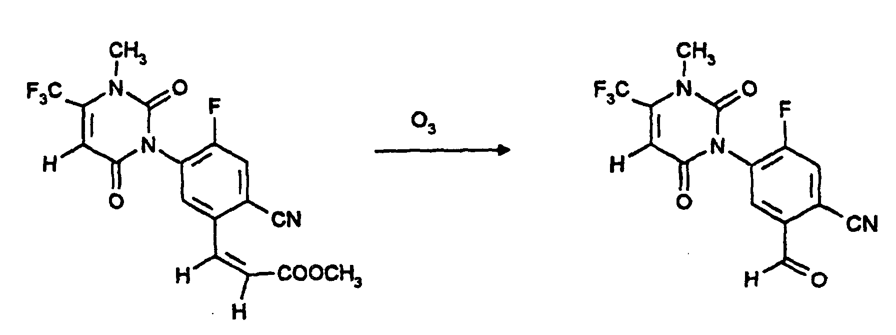

Verwendet man beispielsweise 1-(4-Cyano-2-fluor-5-formyl-phenyl)-3,6-dihydro-2,6-dioxo-3-methyl-4-trifluormethyl-1(2H)-pyrimidin und O-Methyl-hydroxylamin als Ausgangsstoffe, so kann der Reaktionsablauf beim erfindungsgemäßen Verfahren (b) durch das folgende Formelschema skizziert werden:

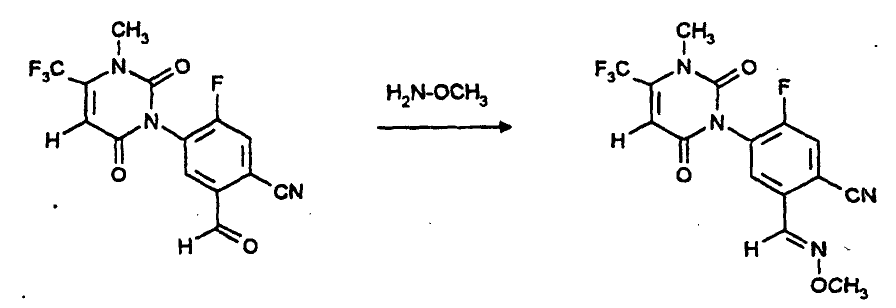

Verwendet man beispielsweise 1-(4-Cyano-2-fluor-5-formyl-phenyl)-3,6-dihydro-2,6-dioxo-3-methyl-4-trifluormethyl-1(2H)-pyrimidin und Methanol als Ausgangsstoffe, so kann der Reaktionsablauf beim erfindungsgemäßen Verfahren (c) durch das folgende Formelschema skizziert werden:

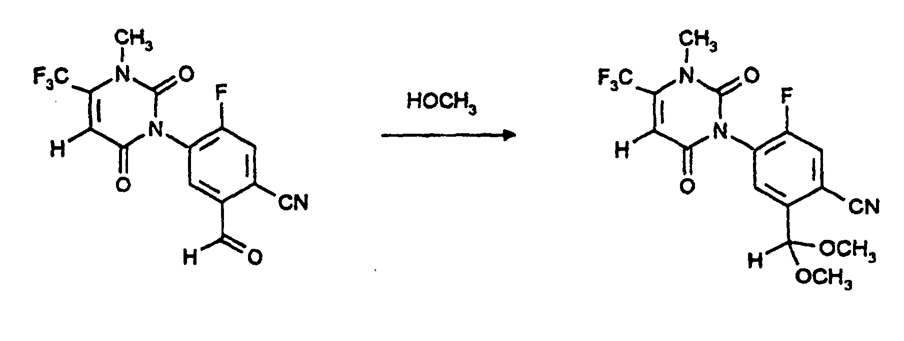

Die beim erfindungsgemäßen Verfahren (a) zur Herstellung der Verbindungen der Formel (I) als Ausgangsstoffe zu verwendenden Alkenylphenyluracile sind durch die Formel (II) allgemein definiert. In der Formel (II) haben R¹, R², R³, R⁴, R⁵ und R⁷ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R¹, R², R³, R⁴, R⁵ und R⁷ angegeben wurde; R¹¹ und R¹² stehen vorzugsweise für Cyano, Carboxy oder C₁-C₄-Alkoxycarbonyl.

Die Ausgangsstoffe der Formel (II) sind noch nicht aus der Literatur bekannt; sie sind als neue Stoffe Gegenstand einer vorgängigen, jedoch nicht vorveröffentlichten Anmeldung (vgl. DE 19 528 186).

Man erhält die Alkenylphenyluracile der allgemeinen Formel (II), wenn man Aminophenyluracile der allgemeinen Formel (V) 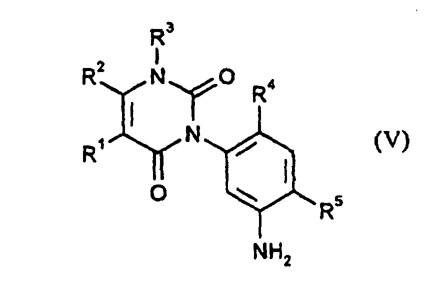 in welcher
- R¹, R², R³, R⁴ und R⁵: die oben angegebenen Bedeutungen haben,
- oder Säureaddukte von Verbindungen der Formel (V) -
mit einem Alkalimetall-nitrit oder Alkyl-nitrit, wie z.B. Natrium-, Kalium-, Methyl-, n- oder t-Butyl-nitrit, und mit einem Hydrogenhalogenid (HX¹), wie z.B. Hydrogenchlorid oder Hydrogenbromid, oder einem Metallhalogenid, wie z.B. Kupfer(I)- oder Kupfer(II)-chlorid, gegebenenfalls in Gegenwart eines Verdünnungsmittels, wie z.B. Wasser, Essigsäure, Acetonitril und Methylenchlorid, bei Temperaturen zwischen -20°C und +10°C umsetzt und die dabei gebildeten Diazoniumsalze der allgemeinen Formel (VI) 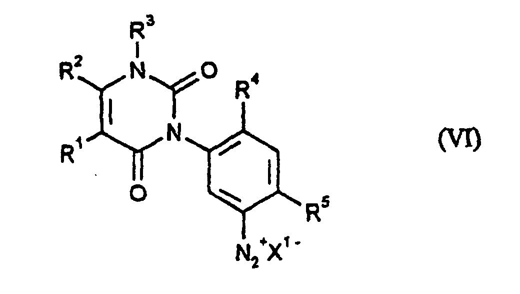 in welcher
- R¹, R², R³, R⁴ und R⁵: die oben angegebenen Bedeutungen haben und
- X¹: für Halogen steht,
mit Acrylsäurederivaten der allgemeinen Formel (VII)

R⁷CH=C(R¹¹,R¹²) (VII)

in welcher
- R⁷, R¹¹ und R¹²: die oben angegebenen Bedeutungen haben,
in Gegenwart von Hydrogenhalogeniden (HX¹), wie z.B. Hydrogenchlorid oder Hydrogenbromid, gegebenenfalls in Gegenwart von Wasser und gegebenenfalls in Gegenwart des anfangs verwendeten organischen Lösungsmittels bei Temperaturen zwischen 0°C und 50°C umsetzt, und die so erhaltenen Produkte mit Säureakzeptoren, wie z.B. Natriumhydrid, in Gegenwart von Verdünnungsmitteln, wie z.B. N,N-Dimethyl-formamid bei Temperaturen zwischen 0°C und 100°C umsetzt (vgl. die Herstellungsbeispiele).

Die bei den erfindungsgemäßen Verfahren (b) und (c) zur Herstellung von Verbindungen der Formel (I) als Ausgangsstoffe zu verwendenden Carbonylphenyluracile sind durch die Formel (Ia) allgemein definiert. In der Formel (Ia) haben R¹, R², R³, R⁴, R⁵ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R², R³, R⁴, R⁵ und R⁷ angegeben wurden.

Die Ausgangsstoffe der Formel (Ia) sind erfindungsgemäße, neue Verbindungen; sie können nach dem erfindungsgemäßen Verfahren (a) hergestellt werden.

Die beim erfindungsgemäßen Verfahren (b) zur Herstellung von Verbindungen der Formel (I) weiter als Ausgangsstoffe zu verwendenden Aminoverbindungen sind durch die Formel (III) allgemein definiert. In der Formel (III) hat R⁸ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁸ angegeben wurde.

Die Ausgangsstoffe der Formel (III) sind bekannte Synthesechemikalien.

Die beim erfindungsgemäßen Verfahren (c) zur Herstellung von Verbindungen der Formel (I) weiter als Ausgangsstoffe zu verwendenden Alkohole oder Mercaptane sind durch die Formeln (IVa) und (IVb) allgemein definiert. In den Formeln (IVa) und (IVb) haben Q¹, Q², R⁹ und R¹⁰ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹, Q², R⁹ und R¹⁰ angegeben wurden.

Die Ausgangsstoffe der Formeln (IVa) und (IVb) sind bekannte Synthesechemikalien.

Als Verdünnungsmittel zur Durchführung der erfindungsgemäßen Verfahren (a) bis (c) kommen vor allem inerte organische Lösungsmittel in Betracht Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren (a) wird in Gegenwart eines Reaktionshilfsmittels durchgeführt. Als Reaktionshilfsmittel kommen hierbei vorzugsweise reduzierend wirkende Chemikalien, wie z.B. Zinkstaub in Essigsäure, Wasserstoff in Gegenwart von Palladium, Natriumdithionit und Dimethylsulfid, in Betracht.

Die Reaktionstemperaturen können bei der Durchführung des erfindungsgemäßen Verfahrens (a) in einem größeren Bereich variiert werden. Im allgemeinen arbeitet man bei Temperaturen zwischen -100°C und +50°C, vorzugsweise zwischen -80°C und +30°C.

Das erfindungsgemäße Verfahren (a) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

In einer bevorzugten Ausführungsform des erfindungsgemäßen Verfahrens (a) wird die Ausgangsverbindung der Formel (II) in einem Verdünnungsmittel vorgelegt und nach Abkühlen auf die erforderliche Temperatur wird Ozon bis zum Ende der Umsetzung eingeleitet. Nach Entfernen des Kühlbades wird das Reaktionshilfsmittel eindosiert und die Mischung bis zum Ende der Umsetzung bei der erforderlichen Temperatur gerührt. Die Aufarbeitung kann auf übliche Weise durchgeführt werden (vgl. die Herstellungsbeispiele).

Als Reaktionshilfsmittel für das erfindungsgemäße Verfahren (b) kommen im allgemeinen die üblichen anorganischen oder organischen Basen oder Säureakzeptoren in Betracht Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methylpiperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren (b) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens (b) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Das erfindungsgemäße Verfahren (c) wird vorzugsweise in Gegenwart eines Reaktionshilfsmittels durchgeführt. Geeignete Reaktionshilfsmittel sind hierbei Oxidationsmittel, wie Wasserstoffperoxid (Hydrogenperoxid) und Säuren, wie z.B. Schwefelsäure.

Die Reaktionstemperaturen können bei der Durchführung des erfindungsgemäßen Verfahrens (c) in einem größeren Bereich variiert werden. Im allgemeinen arbeitet man bei Temperaturen zwischen 0°C und 120°C, vorzugsweise zwischen 10°C und 100°C.

Das erfindungsgemäße Verfahren (c) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

In einer bevorzugten Ausführungsform des erfindungsgemäßen Verfahrens (a) wird das Reaktionshilfsmittel zu einer Mischung aus der Ausgangsverbindung der Formel (Ia) und dem Alkohol oder Mercaptan der Formel (IVa) oder (IVb) gegeben und die Reaktionsmischung wird bis zum Ende der Umsetzung gerührt. Die Aufarbeitung erfolgt nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche, oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr, Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

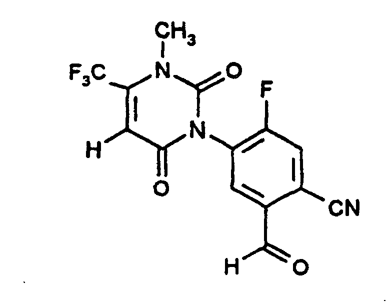

### (Verfahren (a))

In eine auf -70°C abgekühlte Mischung aus 8,7 g (22 mMol) 1-[4-Cyano-2-fluor-5-(2-methoxycarbonyl-ethenyl)-phenyl]-3,6-dihydro-2,6-dioxo-3-methyl-4-trifluormethyl-1(2H)-pyrimidin und 600 ml Methylenchlorid wird 45 Minuten lang Ozon eingeleitet. Dann wird das Kühlbad entfernt und die Mischung mit 10 ml Dimethylsulfid versetzt. Nach Erreichen der Raumtemperatur (ca. 20°C) werden die Phasen getrennt, die organische Phase mit Wasser gewaschen, mit Natriumsulfat getrocknet und filtriert. Das Filtrat wird im Wasserstrahlvakuum eingeengt, der Rückstand mit Diethylether digeriert und das kristalline Produkt durch Absaugen isoliert.

Man erhält 7,4 g (99% der Theorie) 1-(4-Cyano-2-fluor-5-formyl-phenyl)-3,6-dihydro-2,6-dioxo-3-methyl-4-trifluormethyl-1(2H)-pyrimidin vom Schmelzpunkt 177°C.

### Beispiel 2

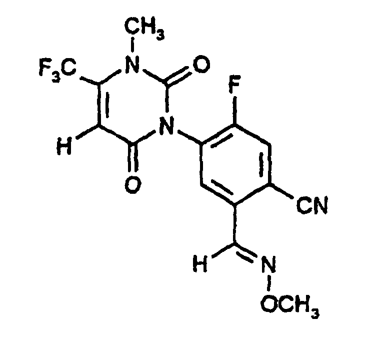

### (Verfahren (b))

Eine Mischung aus 1,0 g (2,9 mMol) 1-(4-Cyano-2-fluor-5-formyl-phenyl)-3,6-dihydro-2,6-dioxo-3-methyl-4-trifluormethyl-1(2H)-pyrimidin, 0,25 g (3,0 mMol) O-Methyl-hydroxylamin-Hydrochlorid, 0,25 g (3,0 mMol) Natriumacetat und 20 ml Ethanol wird ca. 90 Minuten bei Raumtemperatur (ca. 20°C) gerührt. Nach Einengen im Wasserstrahlvakuum wird der Rückstand in Wasser aufgenommen, mit 2N-Salzsäure versetzt und mit Diethylether extrahiert. Die organische Phase wird mit Natriumsulfat getrocknet und filtriert. Vom Filtrat wird dann das Lösungsmittel im Wasserstrahlvakuum sorgfältig abdestilliert.

Man erhält 0,8 g (74% der Theorie) 1-(4-Cyano-2-fluor-5-methoximinomethylphenyl)-3,6-dihydro-2,6-dioxo-3-methyl-4-trifluormethyl-1(2H)-pyrimidin vom Schmelzpunkt 80°C.

### Beispiel 3

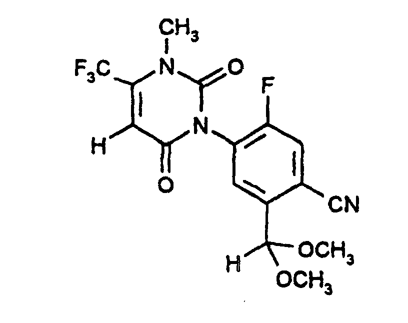

### (Verfahren (c))

0,28 g Wasserstoffperoxid (30%ig) und 0,59 g konz. Schwefelsäure werden bei ca. 10°C vermischt, etwa 2 Stunden bei ca. 20°C gerührt und dann zu einer Mischung aus 1,0 g (2,9 mMol) 1-(4-Cyano-2-fluor-5-formyl-phenyl)-3,6-dihydro-2,6-dioxo-3-methyl-4-trifluormethyl-1(2H)-pyrimidin und 20 ml Methanol gegeben. Die Reaktionsmischung wird ca. 15 Stunden bei Raumtemperatur (ca. 20°C) gerührt und das kristallin anfallende Produkt durch Absaugen isoliert.

Man erhält 0,70 g (62% der Theorie) 1-(4-Cyano-2-fluor-5-dimethoxymethylphenyl)-3,6-dihydro-2,6-dioxo-3-methyl-4-trifluormethyl-1(2H)-pyrimidin vom Schmelzpunkt 164°C.

Analog zu den Herstellungsbeispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

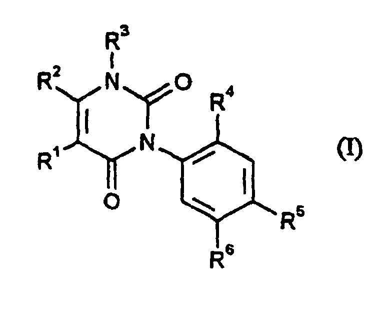

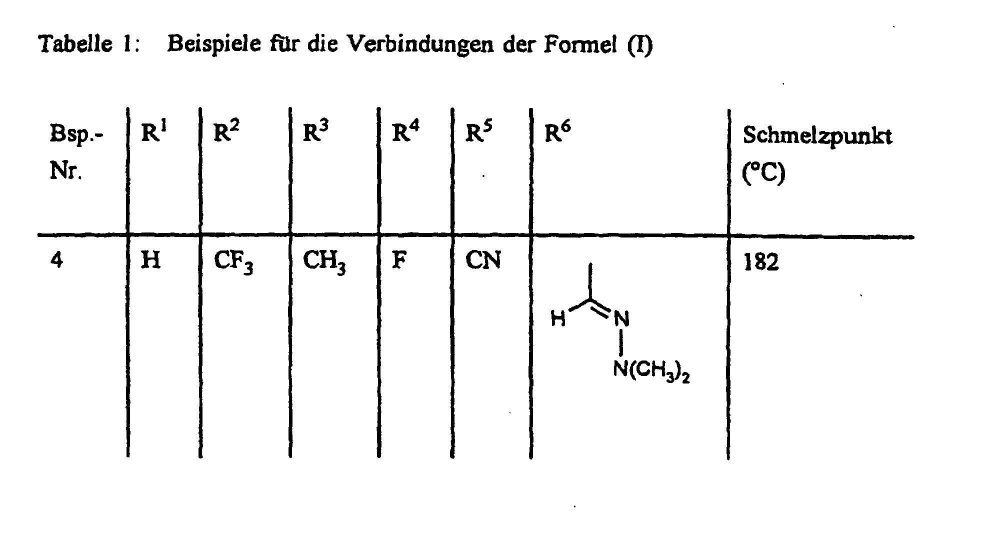

### Ausgangsstoffe der Formel (II):

### Beispiel (II-1)

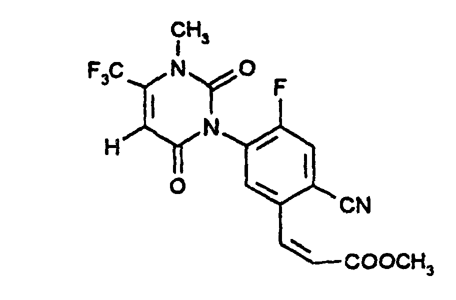

Eine Mischung aus 1,8 g (21 mMol) Acrylsäure-methylester, 1,6 g (15,5 mMol) t-Butylnitrit, 1,6 g (12 mMol) Kupfer(II)-chlorid und 50 ml Acetonitril wird auf ca. 0°C abgekühlt und eine Lösung von 3,3 g (10 mMol) 1-(5-Amino-4-cyano-2-fluorphenyl)-3,6-dihydro-2,6-dioxo-3-methyl-4-trifluormethyl-1(2H)-pyrimidin in 20 ml Acetonitril wird tropfenweise bei dieser Temperatur dazu gegeben. Man lässt dann die Reaktionsmischung auf Raumtemperatur erwärmen und rührt sie 18 Stunden bei dieser Temperatur. Anschließend wird nach Zugabe von 20 ml 1N-Salzsäure mit Essigsäure-ethylester extrahiert, die organische Phase mit Natriumsulfat getrocknet und filtriert. Das Filtrat wird eingeengt und der Rückstand säulenchromatographisch aufgearbeitet. Man erhält 2,8 g (65% der Theorie) 1-[4-Cyano-2-fluor-5-(2-chlor-2-methoxycarbonyl-ethyl)-phenyl]-3,6-dihydro-2,6-dioxo-3-methyl-4-trifluormethyl-1(2H)-pyrimidin vom Schmelzpunkt 46°C.

0,22 g Natriumhydrid (60%ig) werden unter Rühren zu einer auf 0°C abgekühlten Mischung aus 2,0 g (4,6 mMol) 1-[4-Cyano-2-fluor-5-(2-chlor-2-methoxycarbonylethyl)-phenyl]-3,6-dihydro-2,6-dioxo-3-methyl-4-trifluormethyl-1(2H)-pyrimidin und 30 ml N,N-Dimethyl-formamid gegeben und die Reaktionsmischung wird zunächst 15 Minuten bei 0°C, dann ca. 60 Minuten bei 20°C und schließlich 6 Stunden bei 60°C gerührt. Dann wird im Wasserstrahlvakuum eingeengt, der Rückstand mit Diisopropylether verrührt und das kristallin anfallende Produkt durch Absaugen isoliert.

Man erhält 1,1 g (60% der Theorie) 1-[4-Cyano-2-fluor-5-(2-methoxycarbonylethenyl)-phenyl]-3,6-dihydro-2,6-dioxo-3-methyl-4-trifluormethyl-1(2H)-pyrimidin vom Schmelzpunkt 154°C.

### Ausgangsstoffe der Formel (V):

### Beispiel (V-1)

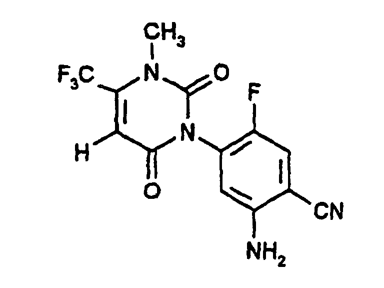

0,17 g (1,2 mMol) Pivalinsäurechlorid werden unter Rühren zu einer Mischung aus 0,50 g (1,2 mMol) 1-(4-Cyano-2-fluor-5-trifluoracetylamino-phenyl)-3,6-dihydro-2,6-dioxo-3-methyl-4-trifluormethyl-1(2H)-pyrimidin, 1 ml Triethylamin und 50 ml Acetonitril gegeben und die Reaktionsmischung wird 18 Stunden bei 20°C und weitere15 Stunden bei 60°C gerührt. Dann wird im Wasserstrahlvakuum eingeengt, der Rückstand mit 1N-Salzsäure/Essigsäureethylester geschüttelt, die organische Phase abgetrennt, mit Natriumsulfat getrocknet und filtriert. Das Filtrat wird im Wasserstrahlvakuum eingeengt und der Rückstand säulenchromatographisch (Kieselgel, Chloroform/Essigsäureethylester, Vol.: 1:1) aufgearbeitet.

Man erhält neben nicht umgesetztem 1-(4-Cyano-2-fluor-5-trifluoracetylaminophenyl)-3,6-dihydro-2,6-dioxo-3-methyl-4-trifluormethyl-1(2H)-pyrimidin (erste Fraktion: 0,30 g) 0,2 g (50% der Theorie) 1-(4-Cyano-2-fluor-5-amino-phenyl)-3,6-dihydro-2,6-dioxo-3-methyl-4-trifluormethyl-1(2H)-pyrimidin als zweite Fraktion.
Schmelzpunkt: 195°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Auf diesen Boden wird nach 24 Stunden die Wirkstoffzubereitung gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen bei Aufwandmengen zwischen 30 und 125 g/ha beispielsweise die Verbindungen gemäß Herstellungsbeispiel 1, 2 und 4 bei guter Verträglichkeit gegenüber Kulturpflanzen, wie z.B. Mais, Gerste und Baumwolle, (0 bis 30 %), sehr starke Wirkung gegen Unkräuter wie Amaranthus (95 %), Sinapis (100 %), Xanthium (100 %), Digitaria (80 bis 100 %), Setaria (95 bis 100 %), Chenopodium (100 %) Matricaria (100 %) und Veronica (100 %).

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0% = keine Wirkung (wie unbehandelte Kontrolle)
100 % = totale Vernichtung

In diesem Test zeigen bei Aufwandmengen zwischen 30 und 125 g/ha beispielsweise die Verbindungen gemäß der Herstellungsbeispiele 1, 2 und 4 bei teilweise guter Verträglichkeit gegenüber Kulturpflanzen, wie z.B. Weizen (10 %), sehr starke Wirkung gegen Unkräuter, wie Abutilon (100 %), Amaranthus (100 %), Galium (100 %), Datura (100 %), Polygonum (100 %) und Solanum (100 %).

## Patentansprüche

1. Substituierte Phenyluracile der allgemeinen Formel (I) 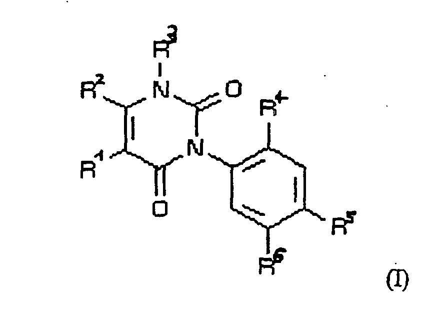 **dadurch gekennzeichnet, daß**
R¹ für Wasserstoff, Fluor, Chlor, Brom oder gegebenenfalls durch Fluor und/oder Chlor substituiertes C₁-C₄-Alkyl steht,
R² für gegebenenfalls durch Fluor und/oder Chlor substituiertes C₁-C₄-Alkyl steht,
R³ für Wasserstoff, Amino, für gegebenenfalls durch Cyano, Fluor, Chlor oder C₁-C₄-Alkoxy substituiertes C₁-C₆-Alkyl oder für gegebenenfalls durch Fluor und/oder Chlor substituiertes C₂-C₆-Alkenyl oder C₂-C₆-Alkinyl steht,
R⁴ für Wasserstoff, Cyano, Fluor oder Chlor steht,
R⁵ für Cyano oder Thiocarbamoyl steht, und
R⁶ für eine der nachstehenden Gruppierungen steht 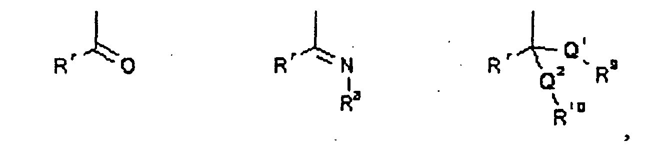
worin
Q¹ und Q² für Sauerstoff oder Schwefel stehen,
R⁷ jeweils für Wasserstoff oder für gegebenenfalls durch Halogen oder C₁-C₄-Alkoxy substituiertes C₁-C₄-Alkyl steht,
R⁸ für Wasserstoff, Hydroxy, Amino, für jeweils gegebenenfalls durch Halogen, C₁-C₄-Alkoxy oder C₁-C₄-Alkoxy-carbonyl substituiertes C₁-C₆-Alkyl, C₁-C₆-Alkoxy, C₁-C₆-Alkylamino, Di-(C₁-C₄-alkyl)-amino, C₁-C₄-Alkylcarbonylamino oder C₁-C₄-Alkyl-sulfonylamino, für jeweils gegebenenfalls durch Halogen substituiertes C₂-C₆-Alkenyl, C₂-C₆-Alkenyloxy oder C₂-C₆-Alkinyl, für jeweils gegebenenfalls durch Halogen oder C₁-C₄-Alkyl substituiertes C₃-C₆-Cycloalkyl, C₃-C₆-Cycloalkyloxy, C₃-C₆-Cycloalkylamino, C₃-C₆-Cycloalkyl-carbonylamino oder C₃-C₆-Cycloalkylsulfonylamino, oder für jeweils gegebenenfalls durch Halogen, C₁-C₄-Alkyl, C₁-C₄-Halogenalkyl, C₁-C₄-Alkoxy oder C₁-C₄-Halogenalkoxy substituiertes Phenyl, Phenoxy, Phenylamino, Phenylcarbonylamino, Phenylsulfonylamino, Phenyl-C₁-C₄-alkyl, Phenyl-C₁-C₄-alkoxy, Phenyl-C₁-C₄-alkylamino steht, sowie
R⁹ und R¹⁰ für C₁-C₄-Alkyl stehen.

2. Substituierte Phenyluracile der allgemeinen Formel (I) gemäß Anspruch 1, **dadurch gekennzeichnet, daß**
R¹ für Wasserstoff, Fluor, Chlor, Brom oder gegebenenfalls durch Fluor und/oder Chlor substituiertes Methyl oder Ethyl steht,
R² für gegebenenfalls durch Fluor und/oder Chlor substituiertes Methyl oder Ethyl steht,
R³ für Wasserstoff, Amino, für gegebenenfalls durch Cyano, Fluor, Chlor, Methoxy oder Ethoxy substituiertes Methyl, Ethyl, n- oder i-Propyl; n-, i-, s- oder t-Butyl, oder für gegebenenfalls durch Fluor und/oder Chlor substituiertes Propenyl, Butenyl, Propinyl oder Butinyl steht,
R⁴ für Wasserstoff, Fluor oder Chlor steht,
R⁵ für Cyano oder Thiocarbamoyl steht, und
R⁶ für eine der nachstehenden Gruppierungen steht 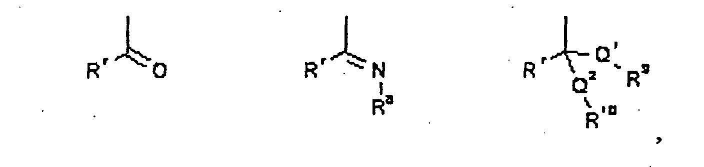
worin
Q¹ und Q² für Sauerstoff oder Schwefel stehen,
R⁷ jeweils für Wasserstoff oder für jeweils gegebenenfalls durch Fluor, Chlor, Methoxy oder Ethoxy substituiertes Methyl oder Ethyl steht,
R⁸ für Wasserstoff, Hydroxy, Amino, für jeweils gegebenenfalls durch Fluor, Chlor, Methoxy, Ethoxy, Methoxycarbonyl oder Ethoxycarbonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Acetylamino, Propionylamino, n- oder i-Butyroylamino, Methylsulfonylamino oder Ethylsulfonylamino, für jeweils gegebenenfalls durch Fluor, Chlor oder Brom substituiertes Propenyl, Butenyl, Propenyloxy, Butenyloxy, Propinyl oder Butinyl, für jeweils gegebenenfalls durch Fluor, Chlor, Brom, Methyl oder Ethyl substituiertes Cyclopentyl, Cyclohexyl, Cyclopentyloxy, Cyclohexyloxy, Cyclopentylamino, Cyclohexylamino, Cyclopropylcarbonylamino, Cyclobutylcarbonylamino, Cyclopentylcarbonylamino, Cyclohexylcarbonylamino, Cyclopropylsulfonylamino, Cyclobutylsulfonylamino, Cyclopentylsulfonylamino oder Cyclohexylsulfonylamino, oder für jeweils gegebenenfalls durch Fluor, Chlor, Brom, Methyl, Ethyl, Trifluormethyl, Methoxy, Ethoxy, Difluormethoxy oder Trifluormethoxy substituiertes Phenyl, Phenylamino, Phenylcarbonylamino, Phenylsulfonylamino, Benzyl, Benzyloxy oder Benzylamino steht, sowie
R⁹ und R¹⁰ für Methyl oder Ethyl stehen.

3. Verfahren zur Herstellung substituierter Phenyluracile der allgemeinen Formel (I) gemäß Anspruch 1, **dadurch gekennzeichnet, daß** man
(a) Alkenylphenyluracile der allgemeinen Formel (II) 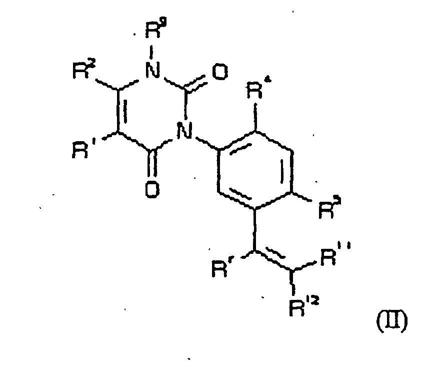 in welcher
R¹, R², R³, R⁴, R⁵ und R⁷ die in Anspruch 1 angegebenen Bedeutungen haben und
R¹¹ und R¹² für Wasserstoff, Cyano, Carboxy oder für C₁-C₄-Alkoxycarbonyl stehen,
mit Ozon umsetzt,
oder daß man
(b) Carbonylphenyluracile der allgemeinen Formel (Ia) 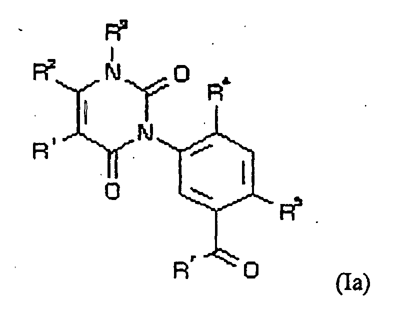 in welcher
R¹, R², R³, R⁴, R⁵ und R⁷ die in Anspruch 1 angegebenen Bedeutungen haben,
mit Aminoverbindungen der allgemeinen Formel (III)
H₂N-R⁸ (III)
in welcher
R⁸ die in Anspruch 1 angegebene Bedeutung hat,
- oder mit Säureaddukten von Verbindungen der Formel (III) - umsetzt,
oder daß man
(c) Carbonylphenyluracile der allgemeinen Formel (Ia) 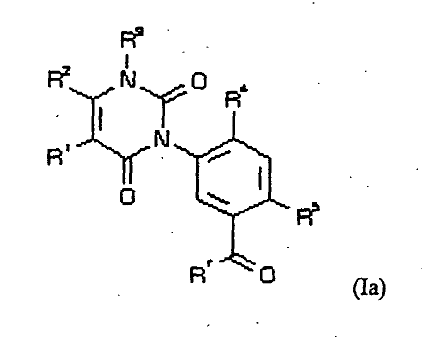 in welcher
R¹, R², R³, R⁴, R⁵ und R⁷ die in Anspruch 1 angegebenen Bedeutungen haben,
mit Alkoholen und/oder Mercaptanen der allgemeinen Formeln (IVa) oder (IVb)
HQ¹R⁹ (IVa)
HQ²R¹⁰ (IVb)
worin
Q¹, Q², R⁹ und R¹⁰ die in Anspruch 1 angegebenen Bedeutungen haben,
umsetzt.

4. Herbizide Mittel, **gekennzeichnet durch** einen Gehalt an mindestens einem Phenyluracil der allgemeinen Formel (I) gemäß dem Anspruch 1.

5. Verfahren zur Bekämpfung von unerwünschten Pflanzen, **dadurch gekennzeichnet, daß** man Phenyluracile der allgemeinen Formel (I) gemäß dem Anspruch 1 auf unerwünschte Pflanzen und/oder ihren Lebensraum einwirken läßt.

6. Verwendung von Phenyluracilen der allgemeinen Formel (I) gemäß dem Anspruch 1 zur Bekämpfung von unerwünschten Pflanzen.

7. Verfahren zur Herstellung von herbiziden Mitteln, **dadurch gekennzeichnet, daß** man Phenyluracile der allgemeinen Formel (I) gemäß dem Anspruch 1 mit Streckmitteln und/oder oberflächenaktiven Substanzen vermischt.

## Claims

1. Substituted phenyluracils of the general formula (I) 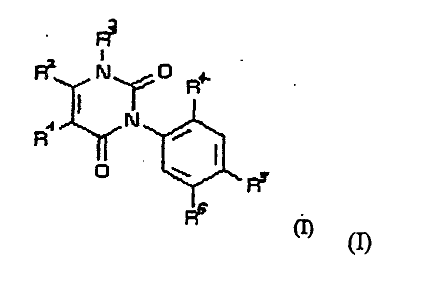 **characterized in that**
R¹ represents hydrogen, fluorine, chlorine, bromine or optionally fluorine- and/or chlorine-substituted C₁-C₄-alkyl,
R² represents optionally fluorine- and/or chlorine-substituted C₁-C₄-alkyl,
R³ represents hydrogen, amino, represents optionally cyano-, fluorine-, chlorine- or C₁-C₄-alkoxy-substituted C₁-C₆-alkyl or represents optionally fluorine- and/or chlorine-substituted C₂-C₆-alkenyl or C₂-C₆-alkinyl,
R⁴ represents hydrogen, cyano, fluorine or chlorine,
R⁵ represents cyano or thiocarbamoyl, and
R⁶ represents one of the groups below 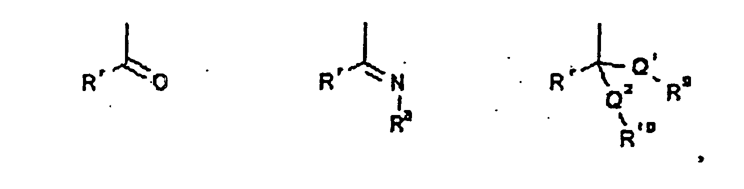
in which
Q¹ and Q² each represent oxygen or sulphur,
R⁷ x in each case represents hydrogen or represents optionally halogen- or C₁-C₄-alkoxy-substituted C₁-C₄-alkyl,
R⁸ represents hydrogen, hydroxyl, amino, represents C₁-C₆-alkyl, C₁-C₆-alkoxy, C₁-C₆-alkylamino, di-(C₁-C₄-alkyl)-amino, C₁-C₄-alkylcarbonylamino or C₁-C₄-alkyl-sulphonylamino, each of which is optionally substituted by halogen, C₁-C₄-alkoxy or C₁-C₄-alkoxy-carbonyl, represents C₂-C₆-alkenyl, C₂-C₆-alkenyloxy or C₂-C₆-alkinyl, each of which is optionally substituted by halogen, represents C₃-C₆-cycloalkyl, C₃-C₆-cycloalkyloxy, C₃-C₆-cycloalkylamino, C₃-C₆-cycloalkyl-carbonylamino or C₃-C₆-cycloalkylsulphonylamino, each of which is optionally substituted by halogen or C₁-C₄-alkyl, or represents phenyl, phenoxy, phenylamino, phenylcarbonylamino, phenylsulphonylamino, phenyl-C₁-C₄-alkyl, phenyl-C₁-C₄-alkoxy, phenyl-C₁-C₄-alkylamino, each of which is optionally substituted by halogen, C₁-C₄-alkyl, C₁-C₄-halogenoalkyl, C₁-C₄-alkoxy or C₁-C₄-halogenoalkoxy, and
R⁹ and R¹⁰ each represent C₁-C₄-alkyl.

2. Substituted phenyluracils of the general formula (I) according to Claim 1, **characterized in that**
R¹ represents hydrogen, fluorine, chlorine, bromine or optionally fluorine- and/or chlorine-substituted methyl or ethyl,
R² represents optionally fluorine- and/or chlorine-substituted methyl or ethyl,
R³ represents hydrogen, amino, represents methyl, ethyl, n- or i-propyl, n-, i-, s- or t-butyl, each of which is optionally substituted by cyano, fluorine, chlorine, methoxy or ethoxy, or represents optionally fluorine- and/or chlorine-substituted propenyl, butenyl, propinyl or butinyl,
R⁴ represents hydrogen, fluorine or chlorine,
R⁵ xrepresents cyano or thiocarbamoyl, and
R⁶ represents one of the groups below 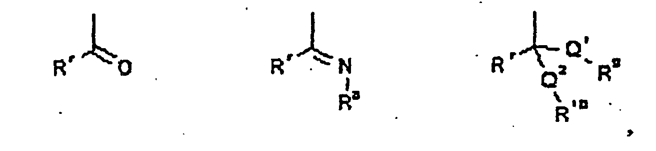
in which
Q¹ and Q² each represent oxygen or sulphur,
R⁷ in each case represents hydrogen or represents methyl or ethyl, each of which is optionally substituted by fluorine, chlorine, methoxy or ethoxy,
R⁸ represents hydrogen, hydroxyl, amino, represents methyl, ethyl, n- or i-propyl, n-, i-, s- or t-butyl, methoxy, ethoxy, n- or i-propoxy, n-, i-, s- or t-butoxy, methylamino, ethylamino, n- or i-propylamino, n-, i-, s- or t-butylamino, dimethylamino, acetylamino, propionylamino, n- or i-butyroylamino, methylsulphonylamino or ethylsulphonylamino, each of which is optionally substituted by fluorine, chlorine, methoxy, ethoxy, methoxycarbonyl or ethoxycarbonyl, represents propenyl, butenyl, propenyloxy, butenyloxy, propinyl or butinyl, each of which is optionally substituted by fluorine, chlorine or bromine, represents cyclopentyl, cyclohexyl, cyclopentyloxy, cyclohexyloxy, cyclopentylamino, cyclohexylamino, cyclopropylcarbonylamino, cyclobutylcarbonylamino, cyclopentylcarbonylamino, cyclohexylcarbonylamino, cyclopropylsulphonylamino, cyclobutylsulphonylamino, cyclopentylsulphonylamino or cyclohexylsulphonylamino, each of which is optionally substituted by fluorine, chlorine, bromine, methyl or ethyl, or represents phenyl, phenylamino, phenylcarbonylamino, phenylsulphonylamino, benzyl, benzyloxy or benzylamino, each of which is optionally substituted by fluorine, chlorine, bromine, methyl, ethyl, trifluoromethyl, methoxy, ethoxy, difluoromethoxy or trifluoromethoxy, and
R⁹ and R¹⁰ each represent methyl or ethyl.

3. Process for preparing substituted phenyluracils of the general formula (I) according to Claim 1, **characterized in that**
(a) alkenylphenyluracils of the general formula (II) 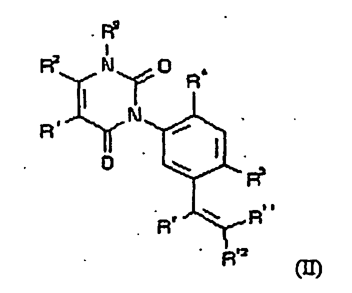 in which
R¹, R², R³, R⁴, R⁵ and R⁷ are each as defined in Claim 1 and
R¹¹ and R¹² each represent hydrogen, cyano, carboxyl or represent C₁-C₄-alkoxycarbonyl,
are reacted with ozone,
or **in that**
(b) carbonylphenyluracils of the general formula (Ia) 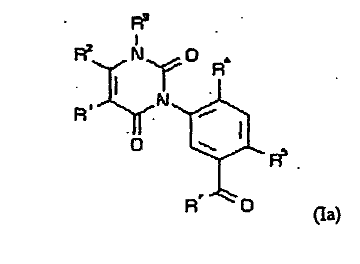 in which
R¹, R², R³, R⁴, R⁵ and R⁷ are each as defined in Claim 1
are reacted with amino compounds of the general formula (III)
H₂N-R⁸ (III)
in which
R⁸ is as defined in Claim 1
- or with acid adducts of compounds of the formula (III) -
or **in that**
(c) carbonylphenyluracils of the general formula (Ia) 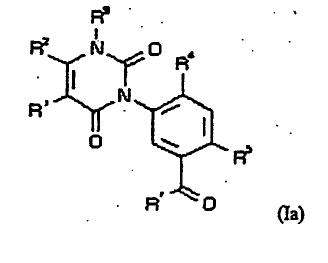 in which
R¹, R², R³, R⁴, R⁵ and R⁷ are each as defined in Claim 1
are reacted with alcohols and/or mercaptans of the general formulae (IVa) or (IVb)
HQ¹R⁹ (IVa)
HQ²R¹⁰ (IVb)
in which
Q¹, Q², R⁹ and R¹⁰ are each as defined in Claim 1.

4. Herbicidal compositions, **characterized in that** they comprise at least one phenyluracil of the general formula (I) according to Claim 1.

5. Method for controlling undesirable plants, **characterized in that** phenyluracils of the general formula (I) according to Claim 1 are allowed to act on undesirable plants and/or their habitat.

6. Use of phenyluracils of the general formula (I) according to Claim 1 for controlling undesirable plants.

7. Process for preparing herbicidal compositions, **characterized in that** phenyluracils of the general formula (I) according to Claim 1 are mixed with extenders and/or surfactants.

## Revendications

1. Phényluraciles substitués de formule générale (I) 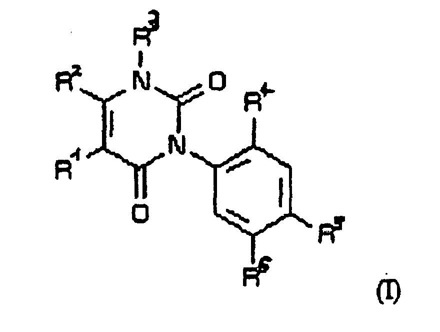 **caractérisés en ce que**
R¹ représente l'hydrogène, le fluor, le chlore, le brome ou un reste alkyle en C₁ à C₄ éventuellement substitué par du fluor et/ou du chlore,
R² est un reste alkyle en C₁ à C₄ éventuellement substitué par du fluor et/ou du chlore,
R³ représente l'hydrogène, un groupe amino, un reste alkyle en C₁ à C₆ éventuellement substitué par un radical cyano, fluoro, chloro ou alkoxy en C₁ à C₄, ou un reste alcényle en C₂ à C₆ ou alcynyle en C₂ à C₆ éventuellement substitué par du fluor et/ou du chlore,
R⁴ représente l'hydrogène, un groupe cyano, le fluor ou le chlore,
R⁵ représente un groupe cyano ou thiocarbamoyle, et
R⁶ représente l'un des groupements suivants 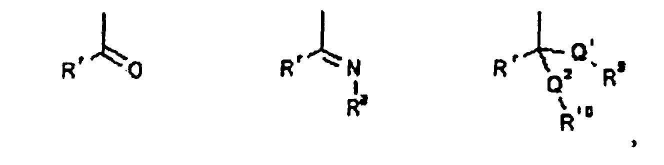
dans lesquels
Q¹ et Q² représentent l'oxygène ou le soufre,
R⁷ représente dans chaque cas l'hydrogène ou un reste alkyle en C₁ à C₄ éventuellement substitué par un halogène ou radical alkoxy en C₁ à C₄,
R⁸ représente l'hydrogène, un groupe hydroxy, amino, un reste alkyle en C₁ à C₆, alkoxy en C₁ à C₆, alkylamino en C₁ à C₆, di (alkyle en C₁ à C₄) amino, (alkyle en C₁ à C₄) carbonylamino ou (alkyle en C₁ à C₄)-sulfonylamino, chacun éventuellement substitué par un radical halogéno, alkoxy en C₁ à C₄ ou (alkoxy en C₁ à C₄)-carbonyle, un reste alcényle en C₂ à C₆, alcényloxy en C₂ à C₆ ou alcynyle en C₂ à C₆, chacun éventuellement substitué par un halogène, un reste cycloalkyle en C₃ à C₆, cycloalkyloxy en C₃ à C₆, cycloalkylamino en C₃ à C₆, (cycloalkyle en C₃ à C₆) carbonylamino, ou (cycloalkyle en C₃ à C₆) sulfonylamino, chacun éventuellement substitué par un halogène ou un radical alkyle en C₁ à C₄, ou bien un reste phényle, phénoxy, phénylamino, phénylcarbonylamino, phénylsulfonylamino, phényl- (alkyle en C₁ à C₄), phényle- (alkoxy en C₁ à C₄), phényl- (alkylamino en C₁ à C₄), chacun éventuellement substitué par un halogène, un radical alkyle en C₁ à C₄, halogénalkyle en C₁ à C₄, alkoxy en C₁ à C₄ ou halogénalkoxy en C₁ à C₄, et
R⁹ et R¹⁰ représentent un reste alkyle en C₁ à C₄.

2. Phényluraciles substitués de formule générale (I) suivant la revendication 1, **caractérisés en ce que**
R¹ représente l'hydrogène, le fluor, le chlore, le brome ou un reste méthyle ou éthyle éventuellement substitué par du fluor et/ou du chlore,
R² est un reste méthyle ou éthyle éventuellement substitué par du fluor et/ou du chlore,
R³ représente l'hydrogène, un groupe amino, un reste méthyle, éthyle, n-propyle, isopropyle, n-butyle, isobutyle, sec.-butyle ou tertiobutyle éventuellement substitué par un radical cyano, fluoro, chloro ou méthoxy ou éthoxy, ou un reste propényle, butényle, propynyle, ou butynyle éventuellement substitué par du fluor et/ou du chlore,
R⁴ représente l'hydrogène, le fluor ou le chlore,
R⁵ représente un groupe cyano ou thiocarbamoyle, et
R⁶ représente l'un des groupements suivants 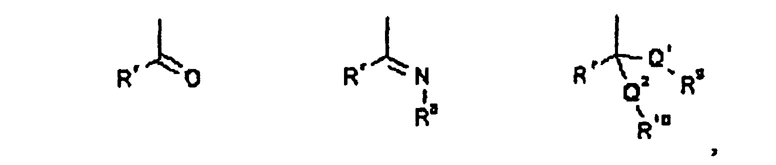
dans lesquels
Q¹ et Q² représentent l'oxygène ou le soufre,
R⁷ représente dans chaque cas l'hydrogène ou un reste méthyle ou éthyle, chacun éventuellement substitué par du fluor, du chlore, un radical méthoxy ou éthoxy,
R⁸ représente l'hydrogène, un groupe hydroxy, amino, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylamino, éthylamino, n-propylamino, isopropylamino, n-butylamino, isobutylamino, sec.-butylamino, tertiobutylamino, diméthylamino, acétylamino, propionylamino, n-butyroylamino, isobutyroylamino, méthylsulfonylamino ou éthylsulfonylamino, chacun éventuellement substitué par un radical fluoro, chloro, méthoxy, éthoxy, méthoxycarbonyle ou éthoxycarbonyle, un reste propényle, butényle, propényloxy, butényloxy, propynyle ou butynyle, chacun éventuellement substitué par du fluor, du chlore ou du brome, un reste cyclopentyle, cyclohexyle, cyclopentyloxy, cyclohexyloxy, cyclopentylamino, cyclohexylamino, cyclopropylcarbonylamino, cyclobutylcarbonylamino, cyclopentylcarbonylamino, cyclohexylcarbonylamino, cyclopropylsulfonylamino, cyclobutylsulfonylamino, cyclopentylsulfonylamino ou cyclohexylsulfonylamino, chacun éventuellement substitué par un radical fluoro, chloro, bromo, méthyle ou éthyle, ou un reste phényle, phénylamino, phénylcarbonylamino, phénylsulfonylamino, benzyle, benzyloxy ou benzylamino, chacun éventuellement substitué par un radical fluoro, chloro, bromo, méthyle, éthyle, trifluorométhyle, méthoxy, éthoxy, difluorométhoxy ou trifluorométhoxy, et
R⁹ et R¹⁰ représentent un groupe méthyle ou éthyle.

3. Procédé de production de phényluraciles substitués de formule générale (I) suivant la revendication 1, **caractérisé en ce que**
(a) on fait réagir avec l'ozone des alcénylphényluraciles de formule générale (II) 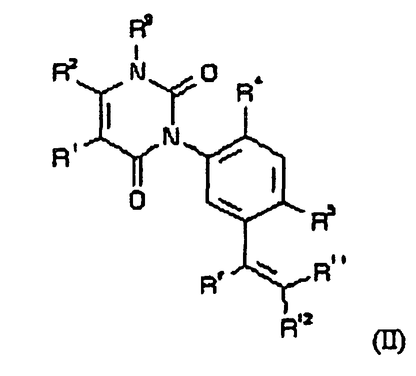 dans laquelle
R¹, R², R³, R⁴, R⁵ et R⁷ ont les définitions indiquées dans la revendication 1
et
R¹¹ et R¹² représentent l'hydrogène, un groupe cyano, carboxy ou un reste (alkoxy en C₁ à C₄)-carbonyle,
ou **en ce que**
(b) on fait réagir des carbonylphényluraciles de formule générale (Ia) 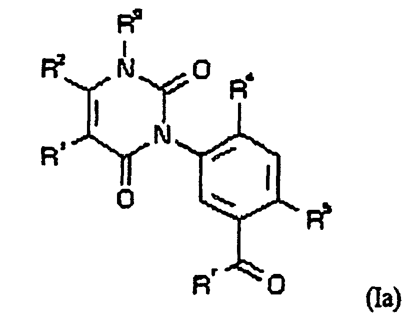 dans laquelle
R¹, R², R³, R⁴, R⁵ et R⁷ ont les définitions indiquées dans la revendication 1,
avec des composés aminés de formule générale (III)
**H**_{**2**}**N-R**^{**8**} (III)
dans laquelle
R⁸ a la définition indiquée dans la revendication 1,
ou avec des produits d'addition d'acides de composés de formule (III),
ou **en ce que**
(c) on fait réagir des carbonylphényluraciles de formule générale (Ia) 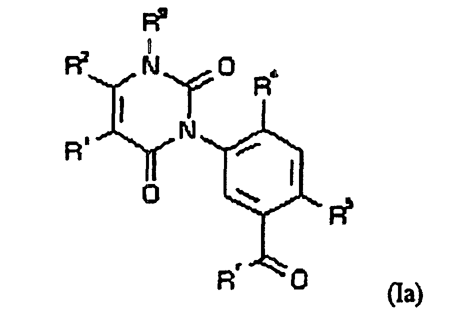 dans laquelle
R¹, R², R³, R⁴, R⁵ et R⁷ ont les définitions indiquées dans la revendication 1,
avec des alcools et/ou des mercaptans de formule générale (Iva) ou (IVb)
**HQ**^{**1**}**R**^{**9**} (IVa)
**HQ**^{**2**}**R**^{**10**} (IVb)
dans lesquelles
Q¹, Q², R⁹ et R¹⁰ ont les définitions indiquées dans la revendication 1.

4. Composition herbicide, **caractérisée par** une teneur en au moins un phényluracile de formule générale (I) suivant la revendication 1.

5. Procédé de lutte contre des plantes indésirables, **caractérisé en ce qu'**on fait agir des phényluraciles de formule générale (I) suivant la revendication 1 sur les plantes indésirables et/ou sur leur milieu.

6. Utilisation de phényluraciles de formule générale (I) suivant la revendication 1 pour combattre des plantes indésirables.

7. Procédé de préparation de compositions herbicides, **caractérisé en ce qu'**on mélange des phényluraciles de formule générale (I) suivant la revendication 1 avec des diluants et/ou des substances tensioactives.
